# EUROPEAN PATENT APPLICATION

(11) **EP 4 725 321 A1**
(43) Date of publication of application: **15.04.2026**
(21) Application number: 25207832.4
(22) Date of filing: 09.10.2025
(51) Int. Cl.: A23L 33/135, A23L 33/175, A61K 31/198, A61K 35/747, A61P 25/16

(54) **PROBIOTIC COCKTAIL MEDICAL FOOD TO ENHANCE L-DOPA STABILITY AND REDUCE SIDE EFFECTS IN PARKINSON'S DISEASE**

(30) Priority: 10.10.2024 US 202463705596 P; 21.02.2025 US 202563761371 P
(71) Applicant: Taipei Medical University, Taipei City 110 (TW)
(72) Inventor: HUANG, HUI-YU, Taipei City 112 (TW)
(74) Representative: Cabinet Chaillot

(57) **Abstract**

The present invention provides a medical food composition comprising at least one probiotic and optionally one or more amino acids. The composition is useful in patients with Parkinson's disease to enhance the systemic stability and central bioavailability of levodopa (L-DOPA). It exerts effects by modulating gut microbiota, reinforcing intestinal barrier integrity, and reducing systemic inflammation and oxidative stress, thereby improving motor and non-motor symptoms and minimizing L-DOPA-induced long-term side effects such as dyskinesia.

## Description

### REFERENCE TO RELATED APPLICATIONS

The present application is based on, claims priority to, and incorporates herein by reference in its entirety for all purposes, U.S. Provisional Application Ser. No. 63/705,596, filed Oct. 10, 2024, and 63/761,371, filed Feb. 21, 2025.

### FIELD OF THE INVENTION

The present invention relates to a probiotic cocktail medical food having a medical adjuvant function, in particular a medical food for improving the stability and mitigating side effects associated with long-term use of L-DOPA treatment in a Parkinson's disease patient. The food comprises at least one probiotic, and optionally amino acid components, which can regulate gut microbiota and improve the metabolic stability of L-DOPA, thereby enhancing the efficacy and mitigating side effects.

### BACKGROUND OF THE INVENTION

Parkinson's disease (PD) is a chronic neurodegenerative disease characterized by the degeneration of central dopamine neurons, commonly seen in the middle-aged and elderly population. Its main clinical symptoms include motor impairments (e.g., tremor, rigidity, slow movements) and non-motor symptoms (e.g., constipation, cognitive disorders, sleep disturbances, etc.).

Nowadays, the first-line drug for the clinical treatment of Parkinson's disease is levodopa (L-DOPA), which is a prodrug of dopamine and can effectively supplement the dopamine content in the brain and significantly improve motor symptoms in patients in a short term. However, L-DOPA treatment presents several well-recognized limitations: (1) its long-term use is prone to cause on-off phenomenon in efficacy; (2) it may give rise to L-DOPA-induced dyskinesia (LID); (3) L-DOPA is rapidly metabolized in the periphery, resulting in a lower effective concentration that actually enters the central nervous system; and (4) its long-term use may also exacerbate non-motor symptoms.

In addition to L-DOPA, the current therapies further include using COMT inhibitors, MAO-B inhibitors, dopamine receptor agonists, and other drugs, but they also suffer from side effects and tolerance issues and cannot effectively address risks of the L-DOPA stability and side effects.

Recent studies have suggested that gut microbiota may take part in the metabolism and absorption of L-DOPA, and that particular species (e.g., *Clostridium and Enterococcus*) will break down L-DOPA prematurely in the gut, reducing its efficiency in entering the blood and brain. Furthermore, intestinal barrier dysfunction and chronic inflammation will exacerbate the PD progression and side effects of treatment.

Therefore, regulating the gut environment and improving microbiota composition and barrier integrity are expected to become inventive directions to improve the stability and safety of L-DOPA. The overall design of an adjuvant therapy for this strategy in the prior art still has shortcomings, especially in terms of enhancing L-DOPA efficacy, stabilizing pharmaceutical metabolism and mitigating side effects. However, there remains a lack of innovative and supportive intervention strategies that are safe, suitable for long-term use, and capable of modulating the gut environment. There is a need to develop an adjuvant therapy that can be administered over a long term, is highly safe and is effective in all ways.

### SUMMARY OF THE INVENTION

The primary purpose of the present invention is to provide a probiotic cocktail medical food (hereinafter referred to as "the Probiotic Cocktail Medical Food", "the composition", or "the formulation") that can slow down the peripheral metabolic rate of L-DOPA in patients having Parkinson's disease, improve its stability and availability in the central nervous system, and further improve the efficacy and mitigate side effects associated with the long-term treatment. The food of the present invention also can improve the stability and integrity of gut microbiota and thus regulates inflammation responses and the nervous function.

For the aforementioned purpose, the present invention proposes a method for alleviating or improving Parkinson's disease symptoms in an individual, comprising administering to the individual a composition comprises at least one probiotic having an intestinal regulatory or neuroprotective function.

For the aforementioned purpose, the present invention proposes a composition for use in a medicament for alleviating or improving Parkinson's disease symptoms, wherein the composition comprises at least one probiotic having an intestinal regulatory or neuroprotective function.

For the aforementioned purpose of the invention, the composition further comprises a functional amino acid combination, wherein the functional amino acid combination comprises four or more essential amino acids.

For the aforementioned purpose of the invention, the use further comprises prolonging the drug stability of an effective drug for Parkinson's disease and reducing side effects of drug.

For the aforementioned purpose of the invention, prolonging the drug stability of the effective drug for Parkinson's disease and reducing side effects of drug refers to increasing the levodopa (L-DOPA) availability in the central nervous system, alleviating L-DOPA-induced dyskinesia (LID), improving non-motor symptoms, reducing inflammation and oxidative stress responses, enhancing the intestinal barrier integrity or the balance of gut microbiota, non-motor symptoms, and improving cognitive symptoms.

For the aforementioned purpose of the invention, the effective drug for Parkinson's disease is selected from the group consisting of levodopa (L-DOPA), a dopaminergic agonist, a MAO-B inhibitor, a DOPA decarboxylase inhibitor, and a COMT inhibitor.

For the aforementioned purpose of the invention, the probiotic is selected from the group consisting of *Lactobacillus, Lactococcus, Streptococcus, Bifidobacterium, Pediococcus,* and *Enterococcus.*

For the aforementioned purpose of the invention, the *Lactobacillus* is selected from the group consisting of *L. acidophilus, L. antri, L. brevis, L. casei, L. coleohominis, L. crispatus, L. curvatus, L. fermentum, L. gasseri, L. johnsonii, L. mucosae, L. pentosus, L. plantarum, L. reuteri, L. rhamnosus, L. sakei, L. salivarius, L. paracasei, L. kisonensis, L. paralimentarius, L. perolens, L. apis, L. ghanensis, L. dextrinicus, L. shenzenensis* and *L. harbinensis.*

For the aforementioned purpose of the invention, the probiotic is a viable bacterium, a dead bacterium, a bacterium sterilized at low temperatures, a lysate, a bacterial supernatant, a cell wall extract, an extracellular polysaccharide or an immunostimulatory component thereof.

For the aforementioned purpose of the invention, the effective drug for the treatment of Parkinson's disease is levodopa (L-DOPA).

For the aforementioned purpose of the invention, the essential amino acid is selected from the group consisting of leucine, isoleucine, valine, lysine, methionine, phenylalanine, tryptophan, threonine, and histidine.

For the aforementioned purpose of the invention, the non-motor symptoms include cognitive disorders, gastrointestinal dysfunction, or sleep disorders.

For the aforementioned purpose of the invention, the composition improves the behavioral function of Parkinson's disease and alleviates L-DOPA-induced dyskinesia through the overall regulatory and neuroprotective functions of the gut-brain axis.

For the aforementioned purpose of the invention, the composition improves the motor and non-motor symptoms of Parkinson's disease and reduces the side effects of L-DOPA by regulating gut microbiota, reducing systemic inflammation and increasing the L-DOPA availability in the central nervous system.

For the aforementioned purpose of the invention, the composition improves the motor and non-motor symptoms of Parkinson's disease and mitigates the side effects of L-DOPA by regulating gut microbiota, reducing systemic inflammation and increasing the L-DOPA availability in the central nervous system.

For the aforementioned purpose of the invention, the Parkinson's disease refers to patients in the prodromal, early or late stages of Parkinson's disease, or who have not received any treatment.

Another purpose of the present invention is to provide a composition for improving Parkinson's disease symptoms, prolonging the drug stability of an effective drug for Parkinson's disease and reducing the side effects of drug, comprising (a) at least one probiotic having intestinal regulatory or neuroprotective functions, and (b) a functional amino acid combination comprising four or more essential amino acids.

For the aforementioned purpose of the invention, the effective drug for the treatment of Parkinson's disease is selected from the group consisting of levodopa (L-DOPA), a dopaminergic agonist, a MAO-B inhibitor, a DOPA decarboxylase inhibitor, and a COMT inhibitor.

For the aforementioned purpose of the invention, the effective drug for the treatment of Parkinson's disease is levodopa (L-DOPA).

For the aforementioned purpose of the invention, the probiotic is selected from *Lactobacillus, Bifidobacterium,* or *Streptococcus.*

For the aforementioned purpose of the invention, the probiotic is a viable bacterium, a dead bacterium, a bacterium sterilized at low temperatures, a lysate, a bacterial supernatant, a cell wall extract, an extracellular polysaccharide, or an immunostimulatory component thereof.

For the aforementioned purpose of the invention, the essential amino acid is selected from the group consisting of leucine, isoleucine, valine, lysine, methionine, phenylalanine, tryptophan, threonine, and histidine.

Another purpose of the present invention is to provide a pharmaceutical composition for improving Parkinson's disease symptoms, comprising (a) an effective drug for the treatment of Parkinson's disease, (b) at least one probiotic having intestinal regulatory or neuroprotective functions, and (c) a functional amino acid combination, wherein the functional amino acid combination comprises four or more than four essential amino acids.

For the aforementioned purpose of the invention, the effective drug for the treatment of Parkinson's disease is levodopa (L-DOPA).

For the aforementioned purpose of the invention, the probiotic is selected from *Lactobacillus, Bifidobacterium,* or *Streptococcus.*

For the aforementioned purpose of the invention, the probiotic is *L. reuteri.*

For the aforementioned purpose of the invention, the probiotic is a viable bacterium, a dead bacterium, a bacterium sterilized at low temperatures, a lysate, a bacterial supernatant, a cell wall extract, an extracellular polysaccharide or an immunostimulatory component thereof.

For the aforementioned purpose of the invention, the essential amino acid is selected from the group consisting of leucine, isoleucine, valine, lysine, methionine, phenylalanine, tryptophan, threonine, and histidine.

For the aforementioned purpose of the invention, the pharmaceutical composition can prolong the drug stability of the effective drug for Parkinson's disease and reduce side effects of drug.

To sum up, the present invention provides a probiotic cocktail medical food with a composition or pharmaceutical composition comprising a probiotic, and a functional amino acid combination as the main components, by virtue of regulatory and neuroprotective mechanisms of the gut-brain axis, which can effectively improve motor and non-motor symptoms in patients having Parkinson's disease and significantly improve the stability and availability of levodopa (L-DOPA) and other therapeutic drugs in the central nervous system. The composition further meets the purpose of delaying the disease progression through reducing inflammation and oxidative stress, enhancing the intestinal barrier function and regulating gut microbiota, in addition to alleviating the side effects of L-DOPA-induced dyskinesia (LID). Compared with the existing therapies that only target a single pharmacological mechanism, the present invention has many advantages in regulating physiological functions, comprises safe composition, is useful for long-term applications, and essentially contributes to improving therapeutic effects on Parkinson's disease and the quality of patients' life, and thus is very promising for industrial application.

### BRIEF DESCRIPTION OF THE DRAWINGS

The techniques of present invention would be more understandable from the detailed description given herein below and the accompanying figures are provided for better illustration, and thus description and figures are not limitative for present invention, and wherein:
FIG. 1 shows an illustration of the experimental animal timeline. It shows the overall procedure of 6-OHDA induction, food administration and behavioral testing (see Example 1).
FIG. 2 shows the immunohistochemistry evaluation of dopaminergic neurons in striatum and substantia nigra pars compacta (SNpc) after treatment for 8 weeks. (A) TH immunostaining images; (B) Quantitative results of TH-positive neurons. One-way ANOVA and Tukey's post hoc test were used for statistical analysis. #p < 0.05 represents a significant difference between Group PD and Group NC, and *p < 0.05 represents a significant difference between Group Y and Group PD.
FIG. 3 shows motor function tests of PD rats. (A) Rotarod test; (B) Grip strength test; (C) Gait test. Data are presented as mean ± SD, and the statistical method is the same as shown in FIG. 2.
FIG. 4 shows non-motor function tests of PD rats. (A) Number of fecal pellets; (B) Fecal moisture content; (C) Performance in Morris water maze; (D-F) Gene expression analysis of tight junction proteins zo1, occ and claudin in the gut.
FIG. 5 shows performance results of (A) inflammatory cytokines IL-6, IL-1β and TNF-α and (B) oxidative stress-related markers (ROS, SOD and GPx) in the rat serum of each group.
FIG. 6 shows the analysis of the relative abundance and diversity of gut microbiota after treatment for 8 weeks. (A) Microbiota distribution at the genus level; (B) α Diversity analysis (Shannon and Simpson indices); (C) Principal coordinate analysis (PCoA) plot.
FIG. 7 shows the bacterial species with significant differences between the groups identified by LEfSe and metaSeq analyses. (A) LEfSe analysis-based heat map; (B) Histogram of significantly altered microbiota identified by metaSeq analysis..
FIG. 8 shows an illustration of the experimental animal timeline (Example 2-1).
FIG. 9 shows the TH immunohistochemistry analysis of dopaminergic neurons in substantia nigra pars (Snpc) after treatment for 8 weeks. (A) TH immunostaining images; (B) Quantitative results of Th-positive cells.
FIG. 10 shows motor function test results of PD rats. (A-B) Rotarod performance; (C) Grip strength test.
FIG. 11 shows the analysis of L-DOPA availability in various treatment groups. (A-C) L-DOPA concentration in serum; (D-F) Dopamine concentration in serum; (G) L-DOPA to DA concentration ratio.
FIG. 12 shows the analysis of colitis-associated gene expression. (A) TLR4; (B) NF-κB; (C) TNF-α; (D) IL-6.
FIG. 13 shows the gene expression analysis of tight junction-associated proteins in the colon. (A) ZO-1; (B) Occludin; (C) Claudin-1.
FIG. 14 shows an illustration of the experimental animal timeline (Example 2-2).
FIG. 15 shows the behavior scoring method and timeline of abnormal involuntary movements (AIMs).
FIG. 16 shows the behavior scoring of abnormal involuntary movements (AIMs scores).
FIG. 17 shows the Morris water maze test for cognitive function analysis. (A) Test representative trajectory diagram; (B) Quantification of test duration; (C) Quantification of the total traveled distance.
FIG. 18 shows the analysis of the stability and metabolism of L-DOPA. (A) L-DOPA concentration; (B) DA concentration; (C) L-DOPA to DA ratio.
FIG. 19 shows the relative abundance of gut microbiota in each group. (A) Firmicutes/Bacteroidota ratio; (B) Microbial composition at the phylum level; (C) Microbial composition at the genus level.
FIG. 20 shows the analysis of bacterial diversity. (A) α-Diversity analysis (Shannon and Simpson indices); (B) Principal coordinate analysis (PCoA) plot.
FIG. 21 shows the results of LEfSe analysis. (A) Bar chart of LDA scores for significantly different taxa; (B) Heat map of microbial composition across different groups.

### DETAILED DESCRIPTION OF THE INVENTION

All technical and scientific terms used in this specification have the meanings commonly understood by those ordinarily skilled in the art unless otherwise defined. The materials used in the present invention are commercially, easily available unless otherwise specified.

The term "a" or "an" does not exclude a plurality, that is to say, unless the context clearly indicates or requires otherwise, the singular forms "a", "an" and "the" should be understood to include plural referents. In other words, unless otherwise expressly stated or the context of reference clearly implies opposite meanings, references to a singular characteristic or limitation throughout the present invention shall include the corresponding a plurality of characteristics or limitations and *vice versa.* Therefore, unless otherwise defined, the terms "a", "an" and "the" have the same meaning as "at least one" or "one or more". For example, reference to "a component" includes mixtures of many components, etc.

The term "about" or "approximately" can compensate for the variability allowed by the pharmaceutical industry and inherent in pharmaceutical products, such as differences in content due to manufacturing variations and/or product degradation over time. This term allows any variation in the actual drug to consider the biological properties of a product under evaluation in a subject equivalent to the enumerated advantages of the claimed product.

The term "composition" refers to a composition of any form, into which many specific components may be optionally incorporated in conjunction with any further components.

The term "comprise" and similar contexts should be interpreted in an open and inclusive manner as "including but not limited to".

The terms "an embodiment", "one embodiment", "a specific embodiment", etc., indicate a unique feature, attribute or characteristic, or a group or set of unique features, attributes or characteristics combined with individual contexts to present in at least one embodiment of the present invention. Such contexts anywhere in this patent specification do not necessarily refer to the same embodiment. Furthermore, the particular features, attributes or characteristics may be combined in any suitable manner in one or more embodiments.

The term "pharmaceutically acceptable" refers to a compound or mixture used in the manufacture of a pharmaceutical composition that is generally safe, non-toxic, and free of undesirable effects in biological and other aspects, including a compound or mixture suitable for human pharmaceutical use.

The term "prevention" refers to preventing a disease, condition, or symptom, as well as preventing further growth and spread of recurrence or progression after the initial improvement or the initial elimination of the cause of the disease, condition, or symptom.

The term "treatment" refers to a therapeutic intervention that can effectively treat a disease, condition or symptom; However, treatment may also refer to improvement, amelioration, control, controlling progression, preventing progression, preventing recurrence, and so on.

The term "probiotic" refers to a microorganism containing viable microbes, that is, capable of reproduction. Probiotic is widely recognized in the state of the art at the time of the patent application as a microorganism that exerts beneficial effects on the host's health when administered in a proper amount.

The term "a probiotic having an intestinal regulatory function" refers to a microorganism that can promote the balance of the intestinal microbial community, enhance the intestinal barrier function, reduce intestinal inflammatory responses or improve gastrointestinal functions, including but not limited to *Lactobacillus, Lactococcus, Streptococcus, Bifidobacterium, Pediococcus,* and *Enterococcus,* etc.

The term "a therapeutically effective dose" refers to a dose sufficient to achieve expected effects of treatment, alleviation, prevention or improvement but not necessarily to eliminate all symptoms completely when a drug is administered to a subject for treating or preventing a target disease (e.g., Parkinson's disease).

The term "neuroprotective function" refers to the physiological effects that can slow down neurodegeneration, reduce neuronal cell death, or promote neuronal repair and remodeling.

The term "L-DOPA-induced dyskinesia (LID)" refers to involuntary, rhythmical or rocking movement symptoms that occur in Parkinson's disease patients after long-term use of L-DOPA, which is one of the common and uncontrollable side effects of drugs.

The term "gut-brain axis" refers to a bidirectional regulation path among gut microbiota, intestines and the central nervous system, relating to multiple mechanisms such as neural, immune and metabolism.

The term "availability in the central nervous system" refers to the extent to which a pharmaceutically active component enters the central nervous system (e.g., the brain). This parameter has a pivotal influence on drug efficacy.

The term "intestinal barrier integrity" refers to the functional integrity of the intestinal epithelial cell layer and tight junctions thereof, capable of effectively prevent harmful substances (e.g., endotoxins, pathogens, etc.) from penetrating into the body's systemic circulation, and maintaining the physiological stability of the intestinal environment and the immune barrier function.

The term "gut microbiota balance" refers to the stable state of the diversity and relative proportion of beneficial bacteria and other symbiotic bacteria communities in the intestine, which has a positive impact on maintaining the health, immune regulation and metabolic function of a host. Microbiota imbalance may be associated with many neurodegenerative diseases, including Parkinson's disease.

The term "non-motor symptoms" refers to other common systemic symptoms in Parkinson's disease patients than motor disorders, including but not limited to emotional disorders, cognitive disorders, autonomic dysfunction (such as gastrointestinal disorders, abnormal urination), sleep disorders, pain and fatigue, etc.

The term "improvement of cognitive symptoms" refers to the improvement of patients' cognitive function performance, including memory, attention, executive function and language comprehension ability, etc. For Parkinson's disease patients, cognitive symptoms often appear during the progression of the disease and greatly impair the quality of life.

The term "essential amino acid" refers to an α-amino acid that cannot be synthesized by the human body or is synthesized at a rate insufficient to meet physiological needs, and thus must be obtained through diet or exogenous supplementation. According to general nutritional definitions, common essential amino acids include leucine, isoleucine, valine, lysine, methionine, phenylalanine, tryptophan, threonine, and histidine.

The present invention relates to a probiotic cocktail medical food for improving or alleviating symptoms of Parkinson's disease, comprising at least one probiotic having an intestinal regulatory or neuroprotective function, and further comprising a functional amino acid combination . The composition can be used as an adjuvant treatment. When used in conjunction with an effective drug for Parkinson's disease, it can increase the drug availability in the central nervous system, prolong the stability of the drug effect, and reduce the related side effects, such as L-DOPA-induced dyskinesia (LID), cognitive dysfunction, gastrointestinal dysfunction, and sleep disorders, etc.

In one embodiment, provided is a composition for alleviating Parkinson's disease symptoms, comprising at least one probiotic having an intestinal regulatory and neuroprotective function. The probiotic can indirectly improve the neural signaling in the brain by regulating gut microbiota and enhancing the intestinal barrier, thereby alleviating the motor or non-motor symptoms in Parkinson's disease patients.

In one embodiment, the composition further comprises a functional amino acid combination.

In one embodiment, the composition can be used in conjunction with an effective drug for Parkinson's disease to improve the stability of drug efficacy and reduce side effects caused by long-term medication. These side effects include L-DOPA-induced dyskinesia (LID), sleep disorders, gastrointestinal dysfunction, and affective disorders.

In one embodiment, the mechanism of the composition includes improving the L-DOPA availability in the central nervous system, delaying the occurrence of LID, reducing inflammation and oxidative stress, improving non-motor symptoms (such as cognitive dysfunction) and maintaining the balance of gut microbiota. These combined mechanisms suggest that the composition holds great promise as a multi-target adjuvant therapy.

In one embodiment, the effective drug for Parkinson's disease is selected from levodopa (L-DOPA), a dopaminergic agonist, a MAO-B inhibitor, a DOPA decarboxylase inhibitor, and a COMT inhibitor and the like.

In one embodiment, the probiotic is selected from any species of *Lactobacillus, Bifidobacterium, Streptococcus, Lactococcus, Pediococcus,* and *Enterococcus,* or mixtures thereof; the bacterium is a viable bacterium, a dead bacterium, a bacterium sterilized at low temperatures, a lysate, an extracellular polysaccharide, or a bacterial supernatant.

In one embodiment, the essential amino acid is selected from the group consisting of leucine, isoleucine, valine, lysine, methionine, phenylalanine, tryptophan, threonine, and histidine.

In one embodiment, the composition can further produce neuroprotective effects by the overall regulation of the gut-brain axis, improve the cognitive and motor function performance, and serve as an adjuvant strategy for long-term L-DOPA treatment to delay the disease progression.

In one embodiment, the composition can be prepared in a form of a powder, a capsule, a liquid or a fermented food according to clinical needs, used as daily healthcare or a drug adjuvant formula, also can be administered in stages in conjunction with drugs to adjust the L-DOPA dosage and reduce the risk of tolerance.

The *Lactobacillus reuteri* Y7 used in this embodiment has been deposited in the Bioresource Conservation and Research Center (BCRC) of Taiwan, under the accession number BCRC 911162. This strain has also been deposited in an international depositary of Japan: The International Patent Organism Depositary, National Institute of Technology and Evaluation (IPOD, NITE) (#120, 2-5-8 Kazusakamatari, Kisarazu-shi, Chiba 292-0818, Japan), under the accession number NITE BP-04078.

### EXAMPLE 1: Efficacy Evaluation of Probiotic Y7 in Improving Motor and Non-motor Disorders in PD

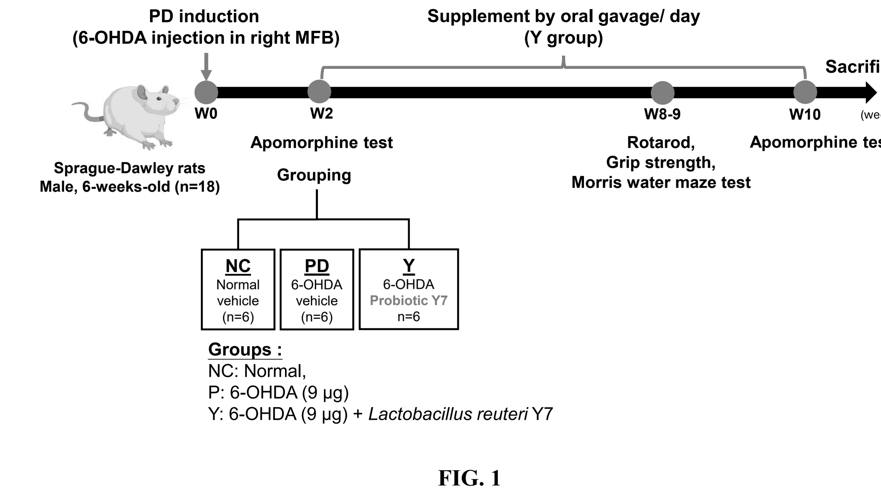

This example is intended to investigate whether the probiotic Y7 can improve motor and non-motor deficiencies in a rat model of Parkinson's disease (PD). The experiment adopted a typical method of preparing a PD model, as shown in FIG. 1, in which the neurotoxin 6-hydroxydopamine (6-OHDA) was injected unilaterally into the medial forebrain bundle (MFB) of rats to induce dopaminergic neuron damage. 18 Male Sprague-Dawley rats were selected and randomly assigned to three groups: normal control group (Group NC), PD model group (Group PD), and probiotic Y7 intervention group (Group Y). After the PD model was built, Group Y was given the probiotic Y7 by oral gavage once daily for 9 consecutive weeks. At weeks 8 and 9 behavioral tests were performed, and tissue and serum samples were collected for analysis.

Two weeks after modeling, rats in the PD model group received an intraperitoneal injection of apomorphine and showed obvious rotation behavior toward the lesion side in an automatic rotation test device, determining that the model was successfully built.

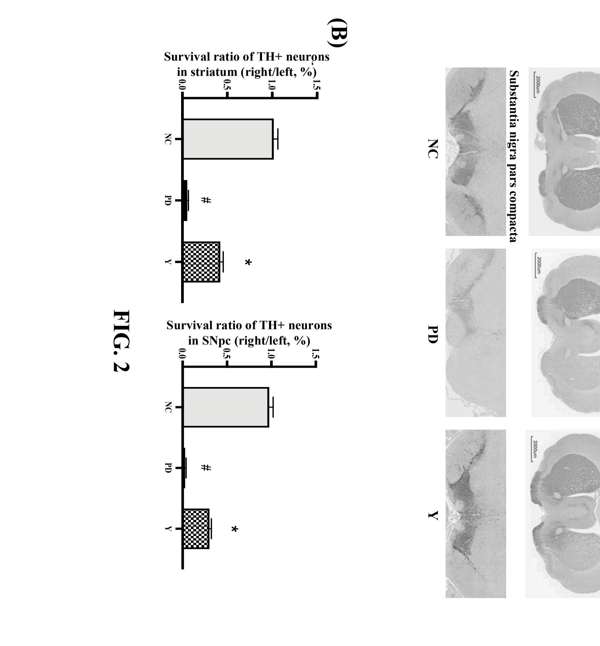

To evaluate the neuroprotective effect on dopaminergic neurons, samples were taken for tyrosine hydroxylase (TH) immunohistochemical staining, and the number of surviving dopaminergic neurons in the striatum and substantia nigra pars compacta (SNpc) was analyzed. The results show that the number of TH-positive neurons in Group PD decreased significantly, while that in Group Y increased significantly (FIG. 2A-2B), indicating that the probiotic Y7 had the potential for neuroprotection.

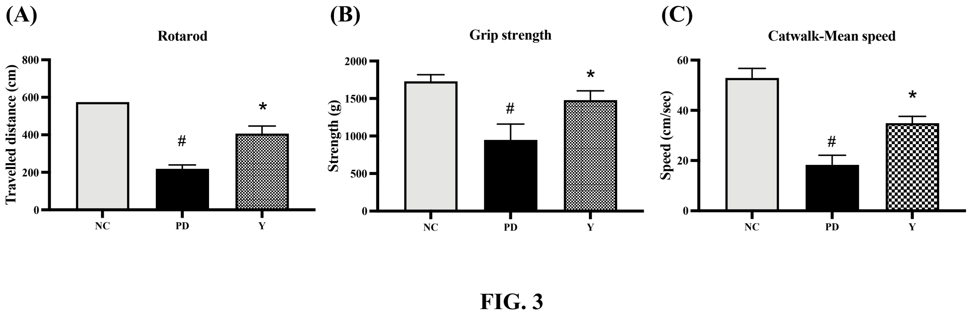

In the aspect of motor function, the Rotarod test results show that Group Y could significantly extend the traveled distance on the rotating rod (FIG. 3A); the muscle strength in the grip strength test was also significantly higher than that of Group PD (FIG. 3B). The gait analysis (Cat Walk) shows that the average walking speed of Group Y was significantly improved (FIG. 3C). It is indicated that the probiotic Y7 can effectively improve motor impairments caused by PD.

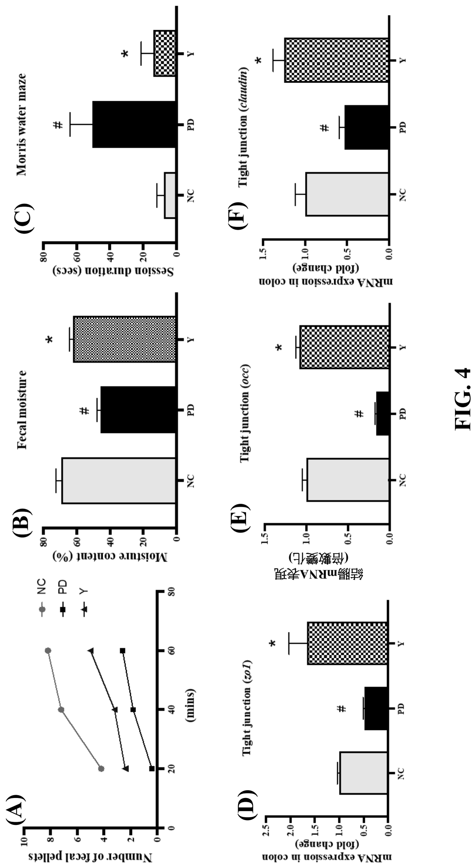

In the aspect of non-motor function, the fecal output and moisture content of rats in Group Y were significantly increased (FIG. 4A-4B), indicating the improvement of intestinal functions. In the Morris water maze test, Group Y had a shortened latency to find the hidden platform, and improved spatial learning and memory abilities (FIG. 4C). In addition, the gene expression of tight junction-associated proteins (zo1, occludin, and claudin) in the rat colon of Group Y was significantly increased (FIG. 4D-4F), indicating that the probiotic Y7 could enhance the intestinal barrier function.

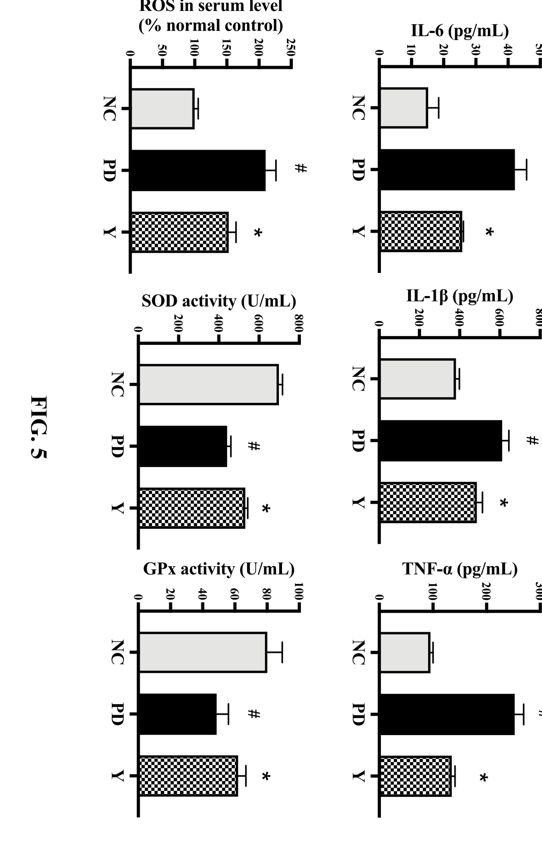

Inflammation and oxidative stress indicators in serum were also improved. Compared with Group PD, the levels of IL-6, IL-1β, and TNF-α in Group Y significantly decreased (FIG. 5A); at the same time, the concentration of reactive oxygen species (ROS) decreased, and the activities of antioxidant enzymes such as superoxide dismutase (SOD) and glutathione peroxidase (GPx) increased significantly (FIG. 5B), indicating that systemic inflammation and oxidative stress were alleviated.

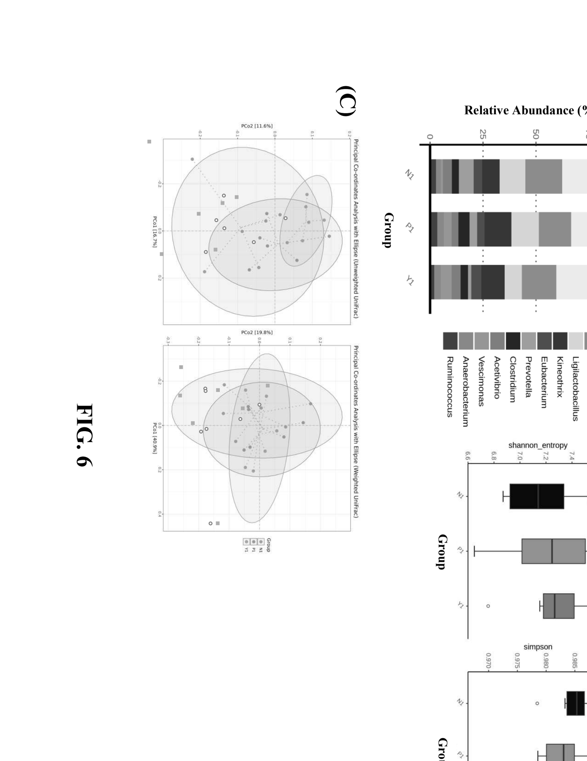

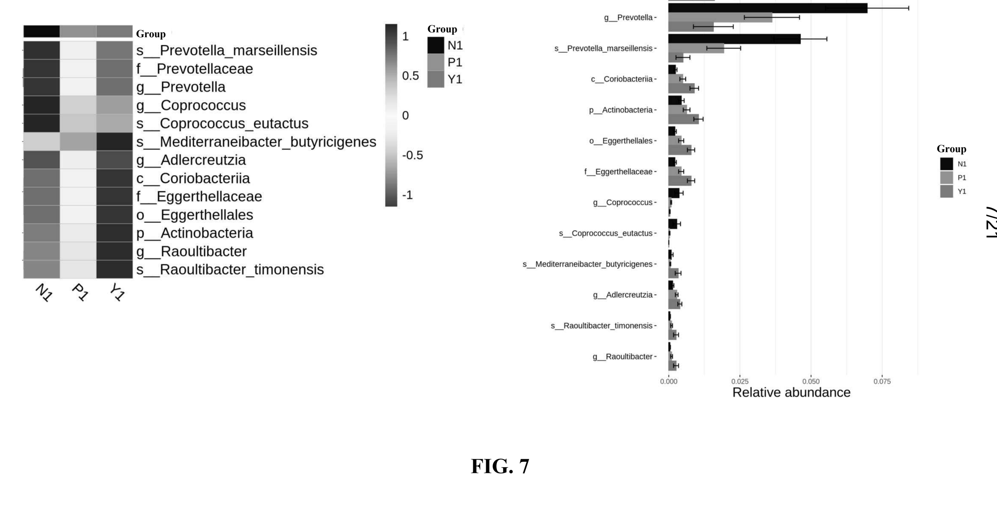

Further analysis of the gut microbiota structure demonstrates that Group Y7 showed changes in relative abundance distribution at the genus level, and presented notable clustering of the microbiota structure in the diversity indices (Shannon, Simpson) and principal coordinate analysis (PCoA) diagram (FIG. 6). Statistical analysis with LEfSe and metaSeq indicates that Y7 could significantly change the composition of dominant and inferior bacteria (FIG. 7), and had the potential to regulate gut microbiota and ecological balance.

Conclusion: The probiotic Y7 can improve both motor and non-motor symptoms of PD rats, and has multiple mechanisms of action, including neuroprotection, anti-inflammation, and regulation of the intestinal barrier and microbiota. It is a gut-brain axis functional composition with the potential for clinical applications.

### Example 2-1: Effects of Probiotic Cocktail Food in Enhancing L-DOPA Therapeutic Stability and Central Neuroprotection

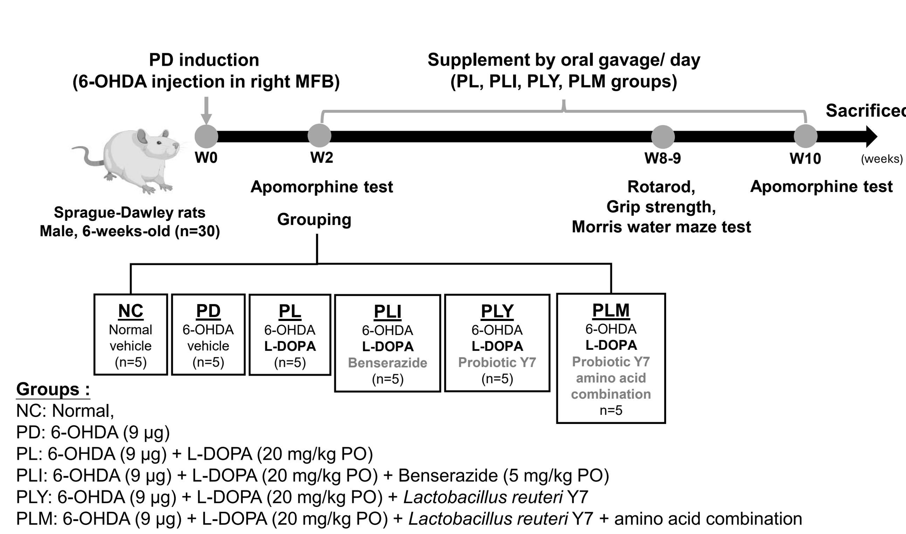

This example is intended to evaluate the adjuvant effect of a probiotic cocktail medical food formulation (hereinafter referred to as "TMU-01 formulation"), which comprises a specific probiotic strain (Lactobacillus reuteri Y7) and a functional amino acid combination, on the treatment of Parkinson's disease (PD), particularly whether it can enhance the stability and availability of L-DOPA in the central nervous system and has a neuroprotective effect. The experiment adopted a typical PD animal model, in which the 6-OHDA was injected unilaterally into the medial forebrain bundle (MFB) of rats to induce dopaminergic neuron damage. 30 Male Sprague-Dawley rats were selected and randomly assigned to six groups: normal control group (Group NC), PD model group (Group PD), L-DOPA treatment group (Group PL), L-DOPA + Benserazide group (Group PLI), L-DOPA + probiotic Y7 group (Group PLY), and L-DOPA + TMU-01 group (Group PLM). Benserazide is an aromatic L-amino acid decarboxylase inhibitor that prevents premature conversion of L-DOPA into dopamine in the periphery, thereby helping improve the efficiency of L-DOPA in entering the central nervous system. All foods were administered by oral gavage once daily for 9 consecutive weeks. Modeling was performed at week 0, behavioral tests were performed at weeks 8 to 9, and sacrifice and sampling were performed at week 10 (FIG. 8).

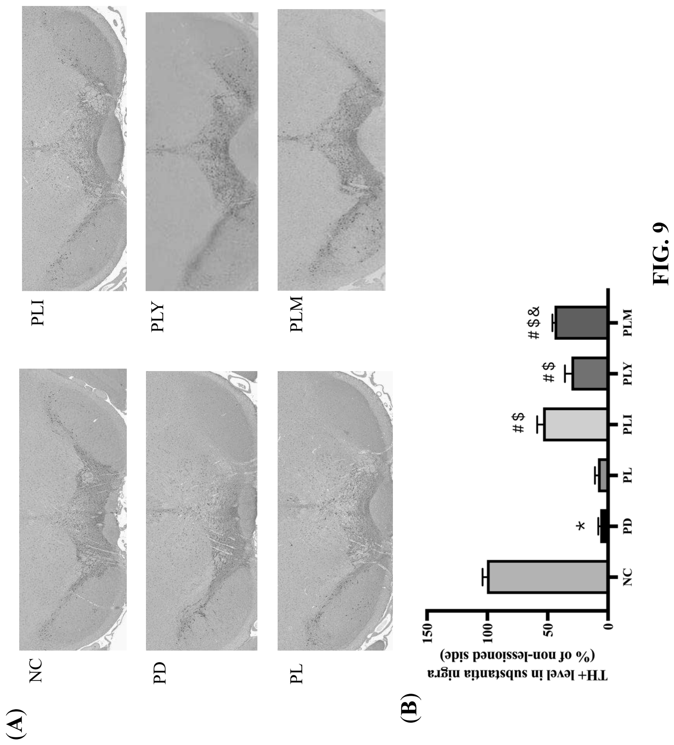

It can be seen in TH immunohistochemical staining that the number of TH-positive neurons in the substantia nigra pars compacta (SNpc) of Group PD was significantly reduced, and only some retained in Group PL. In contrast, the numbers of neurons in Groups PLI, PLY and PLM were significantly restored. Group PLM had the best effect (FIGS. 9A and 9B).

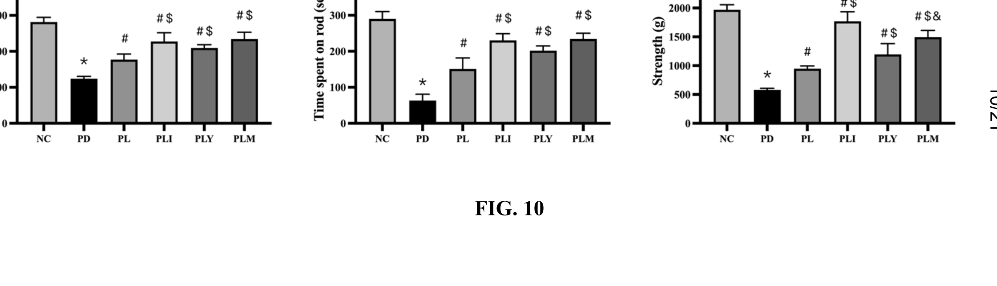

In aspects of motor ability, the Rotarod test shows that the traveled distance and fall time of Groups PLY and PLM were significantly longer than those of Groups PD and PL (FIGS. 10A and 10B); the grip strength test results also show that the muscle strength of Group PLM recovered most significantly (FIG. 10C), indicating that the muscle strength of Group PLM was significantly better than that of the other treatment groups.

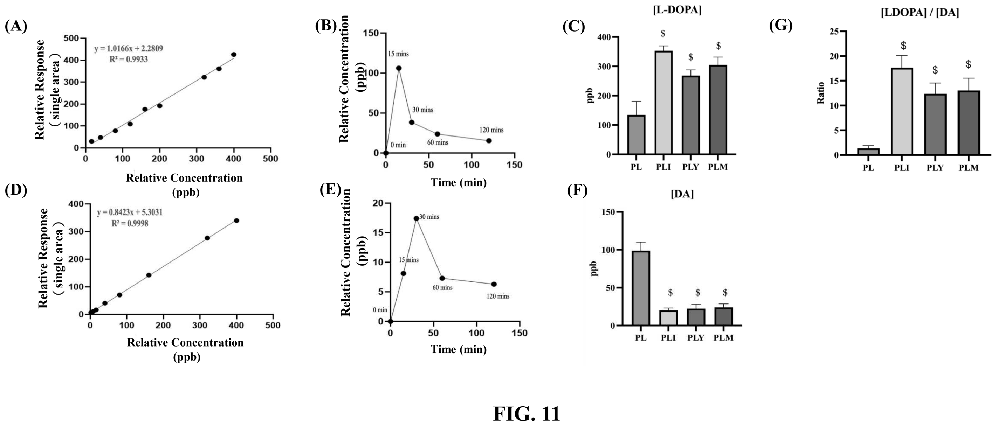

Serum analysis shows that Groups PLI, PLY, and PLM had a significantly higher L-DOPA concentration than Group PL (FIG. 11C), while the DA concentration decreased relatively (FIG. 11F), indicating that the metabolic stability of L-DOPA was improved. The L-DOPA/DA ratio increased in Groups PLI, PLY, and PLM, especially in Group PLM (FIG. 11G), demonstrating that Group PLM had the best availability in the central nervous system.

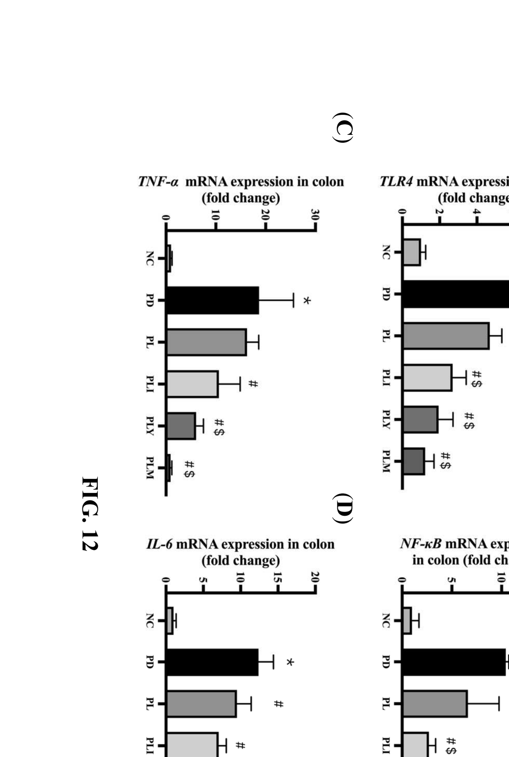

Inflammation-related analysis shows that the expression of inflammatory pathway genes such as *TLR4* (FIG. 12A), *NF-κB* (FIG. 12B), *TNF-α* (FIG. 12C), and *IL-6* (FIG. 12D) in the colon of Group PD rised significantly; while they all showed a downward trend after treatment with PLI, PLY, and PLM, wherein the inhibition was most obvious in Group PLM.

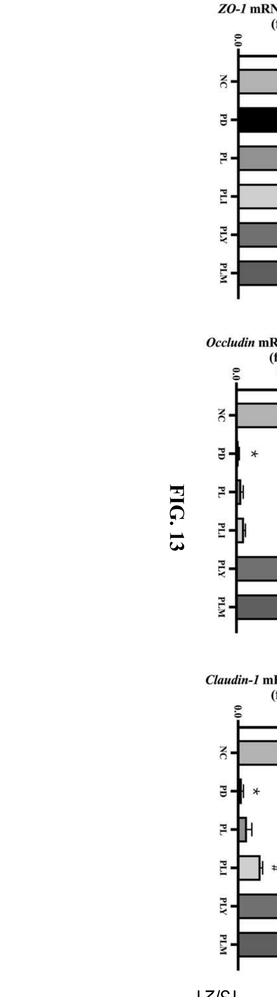

In aspects of the intestinal barrier, Groups PLM and PLY demonstrated a higher gene expression of ZO-1, Occludin, and Claudin-1 (FIGS. 13A-13C), wherein the most notable expression was in Group PLM, indicating a significant improvement in intestinal integrity.

Conclusion: The results of this example demonstrate that the probiotic cocktail medical food can improve the L-DOPA bioavailability, protect neurons, improve the motor performance, inhibit inflammation and enhance the intestinal barrier. Among them, the effects of Group PLM are the most comprehensive, indicating that it can be used as an effective adjuvant regimen for the L-DOPA treatment.

### Example 2-2: Effects of Probiotic Food in Alleviating Side Effects of Long-term L-DOPA Treatment and Improving Microbiota

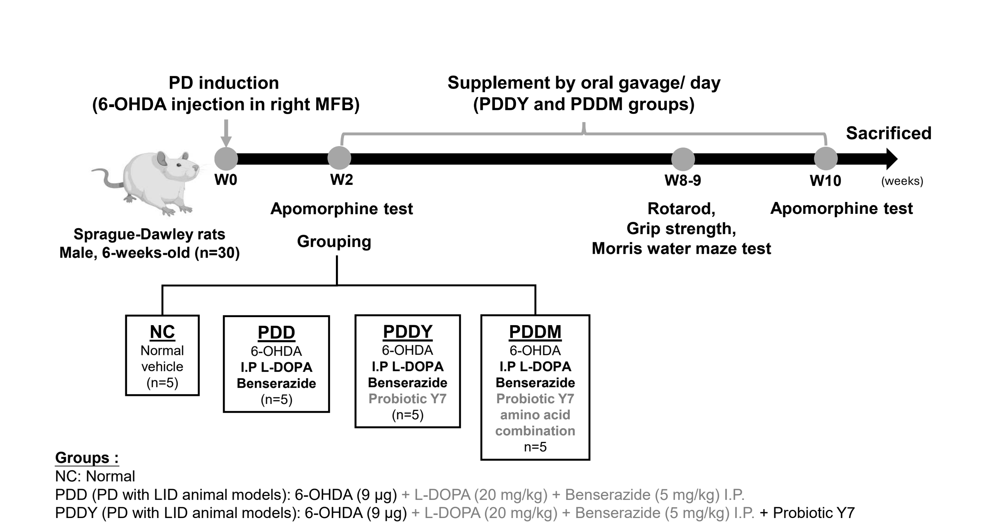

This example is intended to evaluate whether the probiotic cocktail medical food TMU-01 formulation can delay the onset of side effects caused by long-term L-DOPA treatment for Parkinson's disease, and explore the effects on the behavioral performance and the biochemical indicators of the central nervous system. As shown in FIG. 14, the PD rat model was built by the typical method, in which neurotoxin 6-hydroxydopamine (6-OHDA) was injected unilaterally into the medial forebrain bundle (MFB) of rats to induce dopaminergic neuron damage. A total of 30 male Sprague-Dawley rats were selected in the experiment and randomly assigned to four groups: normal control group (NC), PD model + L-DOPA + Benserazide group (PDD), PDD + probiotic Y7 group (PDDY), PDD + TMU-01 group (PDDM), each of which was daily administered by gavage for 9 weeks (FIG. 14).

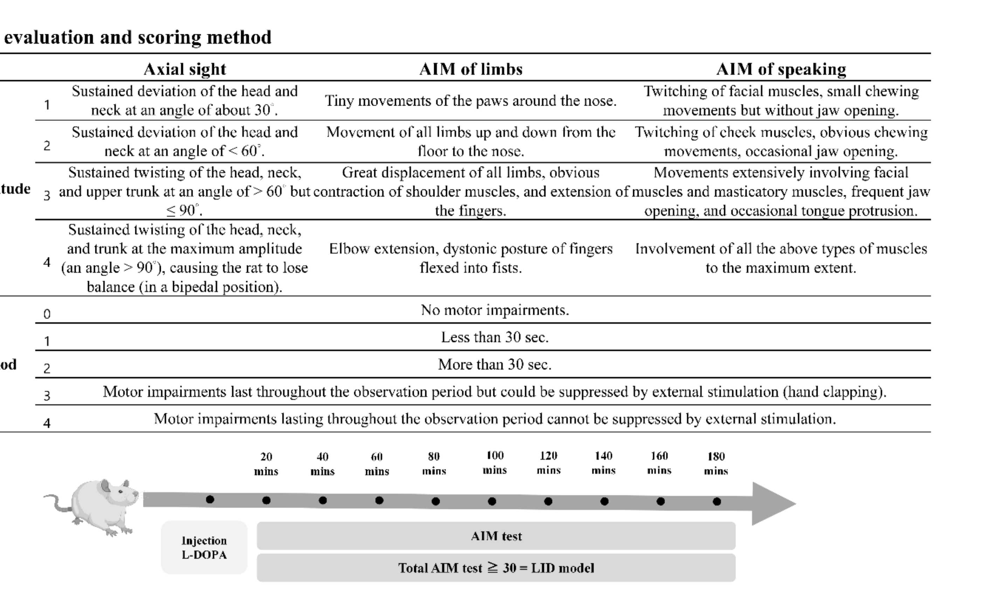

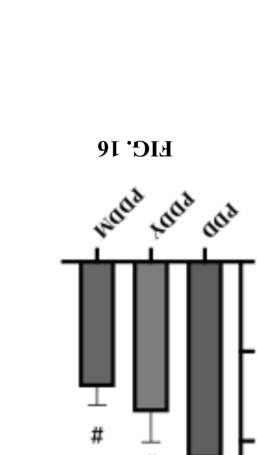

In week 8, the Abnormal Involuntary Movements (AIMs) scoring test was performed, and the behavioral scoring analysis was performed based on the AIMs scoring table (FIG. 15). The experimental results show that the AIMs score of rats in Group PDDM was significantly lower than that of Group PDD treated with L-DOPA + Benserazide alone, indicating that the food could effectively alleviate the side effects induced by L-DOPA (FIG. 16).

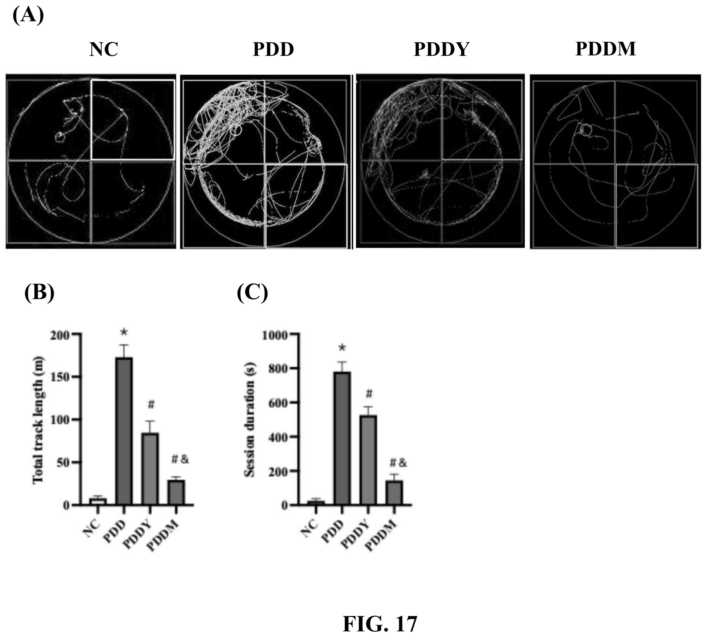

In the Morris water maze test, Group PDDM showed the best performance in both the latency to find the platform and the total swimming distance, indicating that it was superior to other groups in cognitive function performance and had the potential to significantly improve spatial learning and memory (FIGS. 17A-17C).

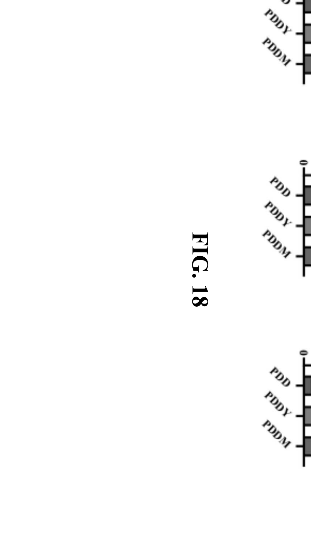

In aspects of L-DOPA metabolic stability, Group PDDM had the highest L-DOPA concentration and L-DOPA/DA ratio in serum (FIGS. 18A-18C), indicating that it could effectively stabilize the in *vivo* L-DOPA concentration and increased the availability to enter the central nervous system.

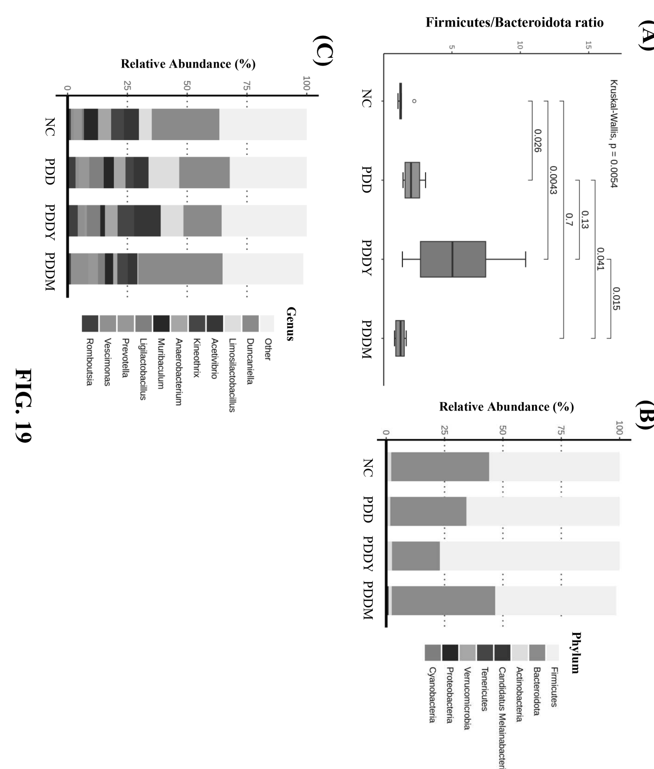

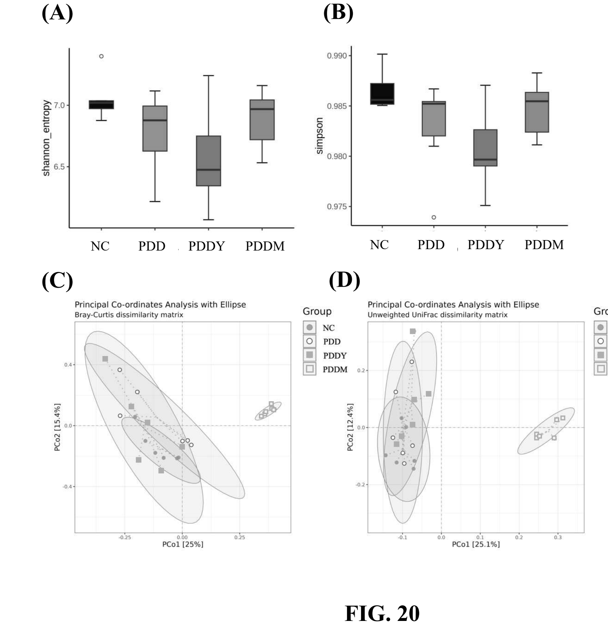

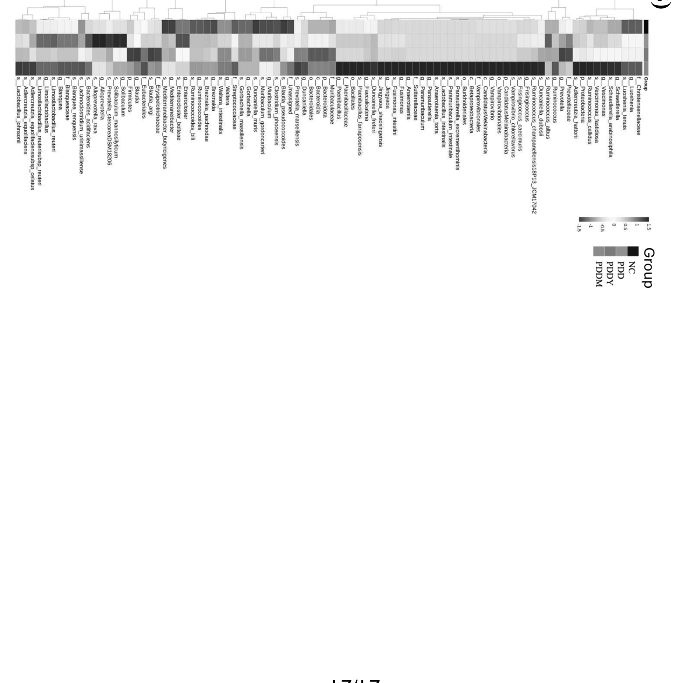

Microbiota analysis shows that Group PDDM had the best performance in aspects of the probiotics recovery at the genus and phylum levels (FIGS. 19A-19C), diversity index (FIG. 20A) and principal coordinate analysis clustering (FIG. 20B). LEfSe and LDA analysis results also support remarkable changes in microbiota characteristics (FIGS. 21A-B). Overall, Group PDDM had the best performance in L-DOPA metabolic stability and gut microbiota regulation, indicating that the TMU-01 formulation had multiple physiological regulation functions and therapeutic potential.

Conclusion: The probiotic cocktail medical food PDDM has a significant therapeutic benefit in the L-DOPA-induced dyskinesia of Parkinson's disease in the rat model. PDDM can effectively alleviate abnormal involuntary movements (AIMs) caused by long-term use of L-DOPA, improve spatial memory and learning ability, and stabilize L-DOPA metabolism to increase its bioavailability. In addition, PDDM can promote the balance of gut microbiota and the probiotics recovery, further supporting its potential as an adjuvant therapy for Parkinson's disease and underlining the importance of intestinal health in the PD treatment.

In summary, the probiotic cocktail medical food TMU-01 formulation provided by the present invention has been demonstrated, by a series of animal experimental results, as a significant and comprehensive adjuvant efficacy in improving Parkinson's disease-related motor and non-motor symptoms and improving the stability and safety of the L-DOPA treatment. The probiotic Y7 alone can improve the motor coordination ability, muscle strength and gait performance of rats in the PD animal model, and also has significant benefits in digestive function, intestinal barrier integrity and spatial memory ability. Moreover, Y7 can effectively inhibit systemic inflammatory responses and oxidative stress, and promote the expression of tight junction proteins and the reconstruction of probiotic microbiota in the intestine, showing that it has an overall regulatory function on the gut-brain axis. Furthermore, when Y7 is used in combination with L-DOPA and a specific amino acid(s) is/are added, it can not only protect the survival of dopaminergic neurons in the central nervous system, but also significantly increase the concentration and stability of L-DOPA in serum, and maintain a high concentration ratio of L-DOPA to dopamine. It is inferred that Y7 has the potential for the promotion of a drug passing the blood-brain barrier and the improvement of drug availability. This probiotic cocktail medical food can also alleviate L-DOPA-induced dyskinesia (LID) caused by long-term L-DOPA use and improve the cognitive impairments. Its effect on alleviating dyskinesia and maze learning performance are better than those of the single drug group.

The inventiveness of the present invention lies in taking intestinal regulation as the core to construct an adjuvant therapy having multi-target and multi-level intervention capabilities by means of probiotics with neuroprotective and anti-inflammatory effects, combined with amino acids that can affect nerve signaling and energy metabolism. Compared with the existing drug designs that only target drug metabolism or receptor effects, the present invention focuses on improving the overall environment of the gut-brain axis, breaking through the cruxes like limited L-DOPA treatment benefits, frequent side effects and insufficient patient tolerance. Therefore, the composition provides a nutritional intervention strategy that is highly safe, can be taken for a long term, and is complementary to and synergistic with the existing L-DOPA therapies. It can be used as an adjuvant treatment for clinical patients having Parkinson's disease or as a preventive solution for healthcare to delay disease progression, reduce drug dosage and improve the quality of life of patients. It has a high practical value and prospects in clinical applications.

Accordingly, it is to be understood that the embodiments of the invention herein described are merely illustrative of the application of the principles of the invention. Reference herein to details of the illustrated embodiments is not intended to limit the scope of the claims, which themselves recite those features regarded as essential to the invention.

### [Biological Material Deposit]

The *Lactobacillus reuteri* Y7 has been deposited in the Bioresource Conservation and Research Center (BCRC) of Taiwan, deposited on November 29, 2022 under the accession number BCRC 911162. The strain has also been deposited in the International Patent Organism Depositary, National Institute of Technology and Evaluation (IPOD, NITE) (#120, 2-5-8 Kazusakamatari, Kisarazu-shi, Chiba 292-0818, Japan), deposited on February 9, 2024 under the accession number NITE BP-04078.

## Claims

1. A composition for use in a medicament for alleviating or improving Parkinson's disease symptoms, wherein the composition comprises at least one probiotic having an intestinal regulatory or neuroprotective function.

2. The composition according to claim 1, wherein the composition further comprises a functional amino acid combination comprising four or more essential amino acids.

3. The composition according to claim 1, wherein the use further comprises prolonging the drug stability of an effective drug for Parkinson's disease and reducing side effects of drug.

4. The composition according to claim 1, wherein the prolonging the drug stability of the effective drug for Parkinson's disease and reducing side effects of drug refers to increasing the levodopa (L-DOPA) availability in the central nervous system, alleviating L-DOPA-induced dyskinesia (LID), improving non-motor symptoms, reducing inflammation and oxidative stress responses, enhancing the intestinal barrier integrity or the balance of gut microbiota, and improving cognitive symptoms.

5. The composition according to claim 3, wherein the effective drug for Parkinson's disease is selected from the group consisting of levodopa (L-DOPA), a dopaminergic agonist, a MAO-B inhibitor, a DOPA decarboxylase inhibitor and a COMT inhibitor.

6. The composition according to claim 1, wherein the probiotic is a viable bacterium, a dead bacterium, a bacterium sterilized at low temperatures, a lysate, a bacterial supernatant, a cell wall extract, an extracellular polysaccharide or an immunostimulatory component thereof.

7. The composition according to claim 3, wherein the effective drug for the treatment of Parkinson's disease is levodopa (L-DOPA).

8. The composition according to claim 1, wherein the composition improves the motor and non-motor symptoms of Parkinson's disease and mitigates the side effects of L-DOPA by regulating gut microbiota, reducing systemic inflammation and increasing the L-DOPA availability in the central nervous system.

9. A composition for improving Parkinson's disease symptoms, prolonging the drug stability of an effective drug for Parkinson's disease and reducing the side effects of drug, comprising (a) at least one probiotic having an intestinal regulatory or neuroprotective function, and (b) a functional amino acid combination comprising four or more essential amino acids.

10. The composition according to claim 9, wherein the effective drug for the treatment of Parkinson's disease is selected from the group consisting of levodopa (L-DOPA), a dopaminergic agonist, a MAO-B inhibitor, a DOPA decarboxylase inhibitor, and a COMT inhibitor.

11. The composition according to claim 9, wherein the effective drug for the treatment of Parkinson's disease is levodopa (L-DOPA).

12. A pharmaceutical composition for improving Parkinson's disease symptoms, comprising (a) an effective drug for the treatment of Parkinson's disease, (b) at least one probiotic having an intestinal regulatory or neuroprotective function, and (c) a functional amino acid combination comprising four or more essential amino acids.

13. The pharmaceutical composition according to claim 12, wherein the effective drug for the treatment of Parkinson's disease is levodopa (L-DOPA).

14. The pharmaceutical composition according to claim 12, wherein the probiotic is *L. reuteri.*

15. The pharmaceutical composition according to claim 12, prolonging the drug stability of the effective drug for Parkinson's disease and reducing side effects of drug.
